# FASCICULE DE BREVET EUROPEEN

(11) **EP 2 035 365 B1**
(45) Date de publication et mention de la délivrance du brevet: **10.04.2013**
(21) Numéro de dépôt: 07788893.1
(22) Date de dépôt: 15.06.2007
(51) Int. Cl.: C07C 209/48, C07C 211/12

(54) **PROCEDE DE FABRICATION DE L´HÈXAMÈTHYLÈNE DIAMINE**
VERFAHREN ZUR HERSTELLUNG VON HEXAMETHYLENDIAMINE
PROCESS FOR THE MANUFACTURE OF HEXAMETHYLEN DIAMIN

(30) Priorité: 20.06.2006 FR 0605464
(43) Date de publication de la demande: 18.03.2009
(73) Titulaire: Rhodia Opérations, 93306 Aubervilliers (FR)
(72) Inventeur: AMOROS, Daniel, 69200 Venissieux (FR); RACHEZ, Denis, 69230 St Genis Laval (FR)
(74) Mandataire: Esson, Jean-Pierre
(86) Numéro de dépôt international: PCT/FR2007/000998
(87) Numéro de publication internationale: WO 2007/147960

(56) Documents cités:
- GB-A- 731 819
- US-A- 3 017 331

## Description

La présente invention concerne un procédé de fabrication d'hexaméthylène diamine par hydrogénation d'adiponitrile en présence d'un catalyseur.

L'hexaméthylène diamine est un intermédiaire chimique de grande importance, notamment utilisé comme monomère dans la fabrication des polyamides. Ainsi, l'hexaméthylène diamine est utilisée en association avec l'acide adipique pour former un sel d'amine, l'adipate d'hexaméthylène diamine, également appelé sel Nylon. Ce sel est mis en oeuvre pour la fabrication de polyadipamide d'hexaméthylène plus usuellement dénommé PA 6,6.

L'hexaméthytène diamine est également un intermédiaire chimique important dans la fabrication des composés diisocyanates.

Les procédés de fabrication d'hexaméthylène diamine utilisés à échelle industrielle, consistent à hydrogéner un composé dinitrile, à savoir l'adiponitrile en présence d'un catalyseur.

Ainsi, plusieurs procédés d'hydrogénation sont connus et exploités. Ils peuvent être classés en deux grands groupes en fonction du catalyseur utilisé et des conditions de température et de pression.

Dans le premier groupe de procédés, le catalyseur est généralement un oxyde métallique tel que l'oxyde de fer ou l'oxyde de cobalt.

L'hydrogénation est réalisée à des pressions et températures élevées et souvent en présence d'ammoniac.

Le second groupe comprend des procédés utilisant des catalyseurs métalliques de type Raney tel que le nickel de Raney ou le cobalt de Raney. L'hydrogénation peut être mise en oeuvre à une pression et une température peu élevées. Le catalyseur métallique de Raney est souvent utilisé en combinaison avec des éléments métalliques ou oxydes métalliques dopant. Pour maintenir l'activité et la sélectivité de ce type de catalyseur, il est nécessaire et obligatoire d'utiliser une base forte.

Dans ces deux groupes de procédés, le produit hydrogéné l'hexaméthylène diamine est récupéré sous forme pure par un enchaînement de distillations destiné à éliminer l'eau et les impuretés légères d'une part et les impuretés lourdes, d'autre part.

Par impuretés légères, on entend, dans le domaine de la distillation de composés organiques, les composés présentant un point d'ébullition inférieur à celui ou ceux de l'hexaméthylène diamine.

De même, les impuretés appelés "lourdes" sont celles qui présentent un point d'ébullition plus élevé que celui de l'hexaméthylène diamine.

Selon les conditions de mise en oeuvre du procédé d'hydrogénation, la formation d'impureté lourdes ou légères sera plus ou moins favorisée ou limitée.

Toutefois, des impuretés se forment obligatoirement car elles sont générées par la décomposition de certains composés ou par réaction entre les molécules présentes.

Parmi ces impuretés, on peut citer l'hexaméthylèneimine (HMI), le diaminocyclohexane (DCH), la tétrahydroazépine (THA), l'aminométhylcyclopentène aminé (AMCPA).

La plupart de ces impuretés sont gênantes dans l'utilisation de l'hexaméthylène diamine notamment comme monomère pour la fabrication des polyamides. En effet, elles peuvent générer des impuretés dans le polyamide obtenu provoquant une coloration jaunâtre de celui-ci et des inhomogénéités dans la matière induisant des défauts et des casses notamment au cours de la fabrication des fils.

La présence de ces impuretés ou composés est notamment détectée et mesurée par analyse polarographique, exprimée quantitativement par l'Indice polarographique (Ipol) ou mole de THA par million de mole de HMD, et par analyse chromatographique phase gaz pour la détermination des concentrations en HMI, DCH et AMCPA.

Il est donc nécessaire de mettre en oeuvre un procédé de récupération de l'hexaméthylène diamine contenu dans le milieu réactionnel d'hydrogénation permettant d'obtenir ce composé avec un degré de pureté élevé.

Il a déjà été proposé un procédé de récupération de l'hexaméthylène diamine comprenant une première distillation du milieu réactionnel d'hydrogénation pour éliminer l'eau. Dans cette colonne de distillation, l'impureté hexaméthylène imine (HMI) est également éliminée car elle est entraînée par l'eau qui distille.

La fraction de queue recueillie dans cette première distillation est soumise à une seconde distillation dans laquelle les impuretés légères sont éliminées.

Le produit hydrogéné pur l'hexaméthylène diamine, est obtenu dans une troisième distillation de la fraction de queue récupérée à la seconde distillation.

Les impuretés lourdes sont éliminées dans la fraction de queue de cette troisième étape de distillation.

Le document US3017331 décrit un tel procédé de purification d'héxaméthylène diamine mettant en oeuvre un réacteur de retenue pour convertir les impuretés légères en impuretés lourdes.

L'enchaînement de distillations décrit ci-dessus présente certains inconvénients. Notamment, il peut être difficile d'obtenir une hexaméthylène diamine présentant une pureté élevée. De plus, la consommation d'énergie est élevée.

Un des buts de la présente invention est de proposer un procédé de fabrication de l'hexamèthylène diamine par hydrogénation de l'adiponitrile comprenant un procédé de récupération de la diamine permettant d'obtenir de manière contrôlée une diamine pure et avec une consommation d'énergie minimisée.

A cet effet, l'invention propose un procédé de fabrication de d'hexaméthylène diamine (HMD) par hydrogénation d'adiponitrile, en présence d'un catalyseur d'hydrogénation consistant à, successivement :
➢ Hydrogéner l'adiponitrile à l'aide d'hydrogène ou d'un gaz contenant de l'hydrogène, et à récupérer ou soutirer de cette étape un flux Eo contenant les composés hydrogénés,
➢ Soumettre le flux Eo à une première distillation pour récupérer une fraction de tête E₁ contenant de l'eau et les imines présentes (HMI), et une fraction de queue Q₁ contenant les composés hydrogénés.
➢ Soumettre le flux de queue Q₁ a une seconde distillation pour récupérer en fraction de queue Q₂ les composés formant des impuretés de point d'ébullition supérieur à celui de HMD appelés impuretés "lourdes", et en fraction de tête E₂ une fraction contenant les composés hydrogénés. Ces impuretés lourdes sont généralement appelées des "goudrons" dans le domaine dé la chimie organique.
> Soumettre le flux de tête E₂ à une troisième distillation pour récupérer une fraction de tête E₃ comprenant les composés ou impuretés légères et une fraction de queue Q₃ contenant les composés hydrogénés et
➢ Soumettre le flux Q₃ a une quatrième distillation pour récupérer une fraction de tête E₄ constituée par HMD et une fraction de queue Q₄ contenant les impuretés lourdes.

Cet enchaînement de distillations successives permet dès la seconde distillation d'éliminer la plus grande partie des composés ou impuretés de température d'ébullition élevée, ou impuretés lourdes.

Ainsi, l'élimination en début de procédé de ces impuretés "lourdes" permet d'améliorer et de faciliter le fonctionnement des distillations suivantes. A titre d'exemple, l'encrassement des éléments de garnissage des colonnes est fortement diminué. En outre, l'énergie nécessaire pour purifier HMD dans les distillations suivantes est minimisée.

Il est également avantageux pour limiter les pertes en hexaméthylène diamine de traiter les fractions Q₂ et Q₄ contenant les impuretés « lourdes ». Ce traitement peut être mis en oeuvre dans une colonne à distiller classique avec distillation de la diamine ou dans des colonnes de type évaporation en couches minces. La diamine récupérée peut être avantageusement recyclée dans une des précédentes colonnes comme la dernière colonne de distillation ou la colonne permettant de séparer les impuretés lourdes et de récupérer la fraction Q₂.

Ce procédé s'applique notamment quand l'étape d'hydrogénation est mise en oeuvre en présence d'un catalyseur à base d'un métal de Raney tel qu'un nickel de Raney ou Cobalt de Raney associé avec une base minérale forte telle que la soude où la potasse.

En effet, la présence d'une base forte peut générer la formation de produits de haut poids moléculaire notamment quand le milieu est chauffé à des températures relativement élevées telles que celles atteintes dans les bouilleurs des colonnes à distiller.

Le procédé de l'invention s'applique à la fabrication de l'hexaméthylène diamine (HMD) par hydrogénation de l'adiponitrile en présence d'un catalyseur à base de nickel de Raney

La réaction d'hydrogénation est mise en oeuvre dans des dispositifs classiques pour la réalisation de cette réaction et à des conditions de température usuelles.

A titre d'exemple d'illustration de mise en oeuvre de cette réaction d'hydrogénation, on peut citer les brevets :FR913997, FR1463409, BE700877, US3821305, US3056837, WO 00/37424 et WO 00/03972.

Les différentes étapes de distillation sont mises en oeuvre dans des dispositifs de distillations classiques et usuels tels que des colonnes à plateaux perforés, colonnes à plateaux à clapet, colonnes à garnissage, colonnes à garnissage structuré, colonne à plateaux.

Les conditions de fonctionnement de ces colonnes seront indiquées dans la description détaillée d'un mode de réalisation de l'invention.

L'invention permet de récupérer une hexaméthylène diamine présentant une pureté élevée répondant aux spécifications requises notamment pour la fabrication de polyamide.

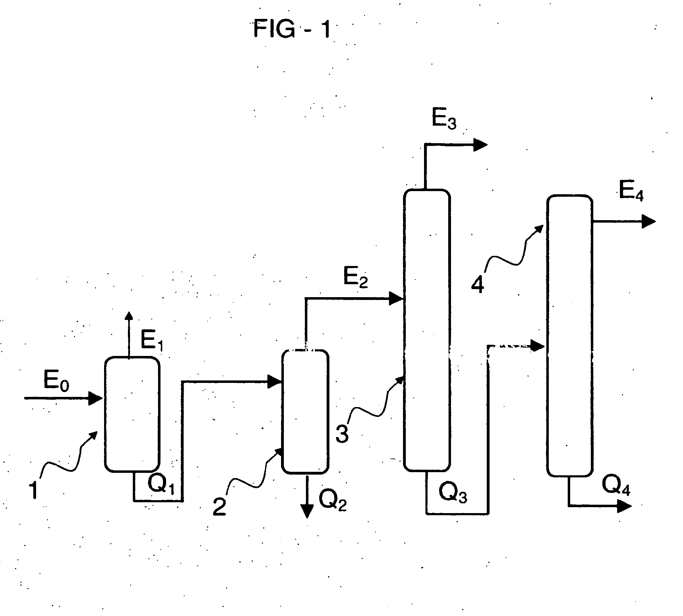

D'autres avantages et détails de l'invention apparaîtront plus clairement au vu de la description détaillée d'un mode de réalisation du procédé de l'invention, en référence à la figure 1 unique annexée représentant un schéma synoptique de ce mode de réalisation du procédé de l'invention.

Un milieu réactionnel Eo provenant d'une étape d'hydrogénation d'adiponitrile, non représentée, en présence d'un catalyseur à base de nickel de Raney et de potasse est alimenté dans une colonne à distiller 1. Cette colonne 1 fonctionne sous une pression de 50 à 220 mm Hg (6.6 kPa àt 29,3kPa) avec un nombre de plateaux théoriques compris entre 5 et 20.

Cette colonne est une colonne à garnissage structuré.

Cette colonne 1 appelée colonne de déshydratation permet de récupérer en tête E₁ l'eau contenu dans le milieu réactionnel ainsi que l'héxaméthylènemine. La fraction de queue Q₁ comprend moins de 50 ppm d'eau.

Cette fraction Q₁ est alimentée dans une deuxième colonne 2 à distiller fonctionnant sous une pression de 50 à 250 mm Hg (6.6 kPa à 33.3 kPa) avec un nombre de plateaux théoriques compris entre 1 et 5.

La fraction de queue Q₂, est constituée de composés de point d'ébullition plus élevé que celui de l'hexaméthylène diamine. Elle représente de 1 à 10% en poids des composés lourds contenus dans le milieu réactionnel E₀.

La fraction de tête E₂ est alimentée dans une troisième colonne à distiller 3 fonctionnant sous une pression dé 10 à 80 mm de Hg (1.3 kPa à 10.6 kPa) avec un nombre de plateaux théorique compris entre 30 et 80. Cette colonne 3 est avantageusement une colonne à garnissage.

La fraction de queue Q₃ récupérée présente une concentration en diaminocyclohexane (DCH) inférieur à 10 ppm.

La fraction de tête E₃ est constituée de composés légers de point d'ébullition inférieur à celui de l'hexaméthylène diamine.

La fraction de queue Q₃ est alimentée dans une quatrième et dernière colonne à distiller 4 fonctionnant sous une pression de 10 à 50 mm Hg (1.3 kPa à 6.6 kPa) et avec un nombre de plateaux théoriques compris entre 30 et 70. Cette colonne 4 est une colonne à plateaux.

L'hexaméthylène diamine est récupérée en tête de colonne sous forme d'un flux E₄ contenant moins de 2 ppm de tétrahydroazépine et un Ipol inférieur à 15

La fraction Q₄ de queue comprend des composés lourds.

Avantageusement, les fractions de queue Q₂ et Q ₄ peuvent être soumises à un traitement pour extraire l'hexaméthylène diamine contenue, par exemple, par une distillation dans une colonne à distiller ou dans une colonne avec évaporation sous forme de couches minces (non représentée).

Le procédé de l'invention en éliminant une partie des composés lourds avant l'élimination des composés légers et la distillation de l'hexaméthylène diamine permet de réaliser une séparation dans les troisième et quatrième colonnes avec un minimum d'énergie et un excellent rendement.

## Revendications

1. Procédé de fabrication d'hexaméthylène diamine par hydrogénation d'adiponitrile en présence d'un catalyseur d'hydrogénation **caractérisé en ce qu'**il consiste à
➢ Hydrogéner l'adiponitrile à l'aide d'hydrogène ou d'un gaz contenant de l'hydrogène.
➢ Soumettre le flux Eo issu du milieu d'hydrogénation à une première distillation, pour récupérer une fraction de tête E₁ contenant l'eau et les imines présentes, et une fraction de queue Q₁ contenant les composés hydrogénés.
➢ Soumettre le flux Q₁ à une seconde distillation pour récupérer une fraction de tête E₂ contenant les composés hydrogénés et une fraction de queue Q₂ contenant des composés de température d'ébullition plus élevé que l'hexaméthylène diamine,
➢ Soumettre la fraction de tête E₂ à une troisième distillation pour récupérer une fraction de queue Q₃ comprenant les composés hydrogénés et une fraction de tête E₃ comprenant les composés de point d'ébullition inférieur à celui de l'hexaméthylène diamine formée, et
➢ Soumettre la fraction de queue Q₃ à une quatrième distillation pour récupérer une fraction de tête E₄ comprenant l'hexaméthylène diamine et une fraction de queue Q₄ contenant les impuretés lourdes.

2. Procédé selon la revendication 1, **caractérisé en ce que** le catalyseur est un métal de Raney choisi dans le groupe comprenant le nickel de Raney, le cobalt de Raney.

3. Procédé selon la revendication 2, **caractérisé en ce que** l'hydrogénation est mis en oeuvre en présence d'une base minérale forte choisie dans le groupe comprenant la soude, la potasse

4. Procédé selon l'une des revendications précédentes **caractérisé en ce que** les étapes de distillation sont réalisées dans des colonnes à distiller choisies dans le groupe comprenant les colonnes à plateaux perforés, les colonnes à plateaux, les colonnes à garnissage, les colonnes à plateaux à clapet, les colonnes à garnissage structuré.

5. Procédé selon l'une des revendications précédentes, **caractérisé en ce que** les fractions contenants des impuretés lourdes, Q₂ et Q₄, sont traitées dans une colonne à distiller pour récupérer l'hexaméthylène diamine et recycler cette hexaméthylène diamine récupérée dans la seconde ou quatrième distillation.

## Claims

1. Process for the manufacture of hexamethylenediamine by hydrogenation of adiponitrile in the presence of a hydrogenation catalyst, **characterized in that** it consists in
➢ Hydrogenating the adiponitrile using hydrogen or a hydrogen-comprising gas.
➢ Subjecting the stream Eₒ resulting from the hydrogenation medium to a first distillation in order to recover a top fraction E₁ comprising the water and the imines present and a bottom fraction Q₁ comprising the hydrogenated compounds.
➢ Subjecting the stream Q₁ to a second distillation in order to recover a top fraction E₂ comprising the hydrogenated compounds and a bottom fraction Q₂ comprising compounds with a higher boiling point than the hexamethylenediamine.
➢ Subjecting the top fraction E₂ to a third distillation in order to recover a bottom fraction Q₃ comprising the hydrogenated compounds and a top fraction E₃ comprising the compounds with a lower boiling point than that of the hexamethylenediamine formed, and
➢ Subjecting the bottom fraction Q₃ to a fourth distillation in order to recover a top fraction E₄ comprising the hexamethylenediamine and a bottom fraction Q₄ comprising the heavy impurities.

2. Process according to Claim 1, **characterized in that** the catalyst is a Raney metal chosen from the group consisting of Raney nickel and Raney cobalt.

3. Process according to Claim 2, **characterized in that** the hydrogenation is carried out in the presence of a strong inorganic base chosen from the group consisting of sodium hydroxide and potassium hydroxide.

4. Process according to one of the preceding claims, **characterized in that** the distillation stages are carried out in distillation columns chosen from the group consisting of perforated plate columns, plate columns, packed columns, valve tray columns and structured packed columns.

5. Process according to one of the preceding claims, **characterized in that** the fractions comprising heavy impurities, Q₂ and Q₄, are treated in a distillation column in order to recover the hexamethylenediamine and to recycle this recovered hexamethylenediamine in the second or fourth distillation.

## Patentansprüche

1. Verfahren zur Herstellung von Hexamethylendiamin durch Hydrierung von Adiponitril in Gegenwart eines Hydrierkatalysators, **dadurch gekennzeichnet, dass** es daraus besteht, dass man
➢ das Adiponitril mit Wasserstoff oder einem Wasserstoff enthaltenden Gas hydriert;
➢ den sich aus dem Hydriermedium ergebenden Strom Eₒ einer ersten Destillation unterwirft, wobei man eine Kopffraktion E₁, die Wasser und die vorhandenen Imine enthält, und eine Sumpffraktion Q₁, die die hydrierten Verbindungen enthält, gewinnt;
➢ den Strom Q₁ einer zweiten Destillation unterwirft, wobei man eine Kopffraktion E₂, die die hydrierten Verbindungen enthält, und eine Sumpffraktion Q₂, die Verbindungen mit einem höheren Siedepunkt als Hexamethylendiamin enthält, gewinnt;
➢ die Kopffraktion E₂ einer dritten Destillation unterwirft, wobei man eine Sumpffraktion Q₃, die die hydrierten Verbindungen enthält, und eine Kopffraktion E₃, die die Verbindungen mit einem niedrigeren Siedepunkt als das gebildete Hexamethylendiamin enthält, gewinnt; und
➢ die Sumpffraktion Q₃ einer vierten Destillation unterwirft, wobei man eine Kopffraktion E₄, die das Hexamethylendiamin enthält, und eine Sumpffraktion Q₄, die die schweren Verunreinigungen enthält, gewinnt.

2. Verfahren nach Anspruch 1, **dadurch gekennzeichnet, dass** es sich bei dem Katalysator um ein Raney-Metall aus der Gruppe bestehend aus Raney-Nickel und Raney-Cobalt handelt.

3. Verfahren nach Anspruch 2, **dadurch gekennzeichnet, dass** man die Hydrierung in Gegenwart einer starken anorganischen Base aus der Gruppe bestehend aus Natriumhydroxid und Kaliumhydroxid durchführt.

4. Verfahren nach einem der vorhergehenden Ansprüche, **dadurch gekennzeichnet, dass** man die Destillationsschritte in Destillationskolonnen aus der Gruppe bestehend aus Lochbodenkolonnen, Bodenkolonnen, Füllkörperkolonnen, Ventilbodenkolonnen und Packungskolonnen durchführt.

5. Verfahren nach einem der vorhergehenden Ansprüche, **dadurch gekennzeichnet, dass** man die schwere Verunreinigungen enthaltenden Fraktionen, Q₂ und Q₄, in einer Destillationskolonne behandelt, wobei man Hexamethylendiamin gewinnt und dieses gewonnene Hexamethylendiamin in die zweite oder vierte Destillation zurückführt.
